Europäisches Patentamt

European Patent Office

Office européen des brevets

(11) Publication number: **0 138 424**
**B1**

(12) **EUROPEAN PATENT SPECIFICATION**

(45) Date of publication of patent specification: **10.05.89**

(21) Application number: **84306456.9**

(22) Date of filing: **21.09.84**

(51) Int. Cl.⁴: **H 01 B 1/20, C 08 L 65/00, H 05 B 3/12, A 61 F 7/08**

(54) Electrical devices comprising conductive polymers exhibiting ptc characteristics.

(30) Priority: **22.09.83 US 535449**
**22.09.83 US 534913**

(43) Date of publication of application:
**24.04.85 Bulletin 85/17**

(45) Publication of the grant of the patent:
**10.05.89 Bulletin 89/19**

(84) Designated Contracting States:
**AT BE CH DE FR GB IT LI NL SE**

(56) References cited:
**DE-A-1 640 842**
**GB-A-1 503 387**
**US-A-3 732 338**
**US-A-4 265 789**

**KURT F. HEINISCH: "Kautschuk-Lexikon", 2nd edition, 1977, pages 432-433 A.W. Genter Verlag, Stuttgart, DE**

(73) Proprietor: **RAYCHEM CORPORATION (a Delaware corporation)**
**300 Constitution Drive**
**Menlo Park, California 94025 (US)**

(72) Inventor: **Cheng, Tai Chun**
**3375 Kenzo Court**
**Mountain View California 94040 (US)**
Inventor: **McKinley, Bruce Alan**
**320 Torrano Common**
**Fremont California 94536 (US)**

(74) Representative: **Jones, David Colin et al**
**Raychem Limited**
**Intellectual Property Law Department**
**Faraday Road Dorcan Swindon**
**Wiltshire SN3 5HH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# EP 0 138 424 B1

**Description**

This invention relates to electrical devices comprising conductive polymer compositions which exhibit PTC behaviour.

Introduction to the Invention

It is known that polymers, including cyrstalline polymers, can be made electrically conductive by dispersing therein suitable amounts of carbon black or another finely divided conductive filler. Some conductive polymers exhibit what is known as PTC (positive temperature coefficient) behavior. The terms "composition exhibiting PTC behavior" and "PTC composition" are used in this specification to denote a composition which preferably has an $R_{14}$ value of at least 2.5 and an $R_{100}$ value of at least 10, particularly an $R_{30}$ value of at least 6, where $R_{14}$ is the ratio of the resistivities at the end and the beginning of a 14°C range, $R_{100}$ is the ratio of the resistivities at the end and the beginning of a 100° range, and $R_{30}$ is the ratio of the resistivites at the end and the beginning of a 30°C range. A plot of the log of the resistance of PTC element (i.e. an element composed of PTC composition) against temperature will often show a sharp change in slope over a part of the temperature range in which the composition has an $R_{100}$ value of at least 10. The term "switching temperature" (usually abreviated to $T_s$) is used herein to denote the temperature at the intersection point of extensions of the substantially striaght portions of such a plot which lie either side of the portions showing the sharp change in slope.

Electrical devices comprising conductive polymer elements, in particular heaters, circuit control devices, and sensors, have been described in prior publications and in co-pending, commonly assigned, patent applications. Reference may be made for example to U.S. Patents Nos. 2,952,761, 2,978,665, 3,243,753, 3,351,882, 3,571,777, 3,757,086, 3,793,716, 3,823,217, 3,858,144, 3,861,029, 3,950,604, 4,017,715, 4,072,848, 4,085,286, 4,117,312, 4,177,376, 4,177,466, 4,188,276, 4,237,441, 4,242,573, 4,246,468, 4,250,400, 4,252,692, 4,255,698, 4,271,350, 4,272,471, 4,304,987, 4,309,596, 4,309,597, 4,314,230, 4,314,231, 4,315,237, 4,317,027, 4,318,881, 4,329,551, 4,330,704, 4,334,351, 4,352,083, 4,361,799, 4,388,607, 4,398,084, 4,413,301, 4,425,397, 4,426,339, 4,426,633, 4,427,877, 4,435,639, 4,429,216 and 4,442,139; J. Applied Polymer Science 19, 813—815 (1975), Klason and Kubat; Polymer Engineering and Science 18, 649—653 (1978), Narkis et al; German OLS 2,634,999, 2,746,602, 2,821,799, and European Application Publication Nos. 38,713, 38,714, 38,718, 63,440, 67,679, 68,688, 74,281, 87,884, 92,406, 96,492, 123,540, 119,807, 133,748 and 136,039.

However, non of the known compositions offer a satisfactoiry combination of physical and electrical properties when a relatively low switching temperature, e.g. 0° to 75°C is required.

US—A—3,732,338, GB—A—1,503,387, DE—A—1,640,842 and US—A—4,265,789 describe various hydrocarbon compositions that may contain carbon black and/or other additives, for the manufacture of products by moulding or extrusion. US—A—3,732,338 describes a mixture of ethylene-propylene rubber, a polypentenamer and a poly (cycloolefin) for use in automobile tyres or hoses. GB—A—1,503,387 describes a vulcanisable moulding composition containing a polyocetenamer. DE—A—1,640,842 describes a cable having a polyolefin conductive intermediate layer of a specified hydrocarbon formulation. US—A—4,265,789 describes an extrudable or mouldable composition comprising conductive particles, such as carbon black, dispersed in a two-phase high molecular weight polymer matrix consisting for example of polyolefindiene and a polyolefin.

Summary of the Invention

We have now discovered that conductive polymer compositions having very valuable properties for electrical devices are those wherein a particulate conductive filler, particularly carbon black, is dispersed in a polymeric component, which component comprises a substantial proportion of a crystalline cylcoolefin polymer. In particular, we have found that such compositions exhibit PTC behavior with a $T_s$ which corresponds to (and generally is 5° to 100°C below, eg 10° to 40°C below) the crystalline melting point of the cycloolefin polymer, which is generally in the range of 0° to 80°C. Especially when the polymer has a melting point below ambient temperature and/or when it is important that the composition has good physical properties, especially flexibility, it is desirable or essential that the composition is cross-linked.

In accordance with the present invention there is provided an electrical device which comprises (1) at PTC element which is composed of a conductive polymer composition which exhibits PTC behavior and which comprises (a) a polymer component which comprises at least 15% by weight of repeating units derived from a cycloolefin, which has a crystallinity of at least 5% as measured by differential scanning calori metry using high density polyethylene as the standard, and which has a crystalline melting point in the range of 0° to 80°C, and (b) a particulate conductive filler which is dispersed in said polymer component; and (2) at least two electrodes which can be connected to a source of electrical power to cause current to pass through the PTC element.

We have further discovered that controlled, safe and economical heating of a human beings and other heat-sensitive substrates can be achieved through use of a self-regulating conductive polymer heater, comprising the electrical device according to the invention, whose maximum surface temperature is less than 45°C, preferably less than 40°C, for example 25 to 45°C, particularly 35 to 40°C. Accordingly, a heat-sensitive substrate may be heated by bringing the substrate into conductive contact with such a heater.

2

# EP 0 138 424 B1

Detailed Description of the Invention

The PTC compositions used in the electrical device of this invention preferably comprise a polymeric component which

(a) comprises at leat 15%, generally at least 20%, preferably at least 50%, especially 50 to 90%, by weight of units derived from a substituted or unsubstituted cycloolefin, preferably a cycloolefin containing 5 to 12 carbon atoms in the ring;

(b) has a crystallinity (crystallinities given in this specification are measured by differential scanning calorimetry (DSC) using high density polyethylene as the standard) of at least 5%, preferably at least 8% (with values of 8—15% being generally preferred for heaters and values above 15% being generally preferred for circuit protection devices); and

(c) has a crystalline melting point of 0° to 80°C, preferably 25° to 70°C.

The cycloolefin units can be derived from a single cycloolfein or from a mixture of two or more cycloolefins. Polymerization of cycloolefins generally results in repeating units which are ethylenically unsaturated, and cycloolefin polymers are, therefore, often referred to as polyalkenamers. The cyclo-olefin units are preferably present as a polymer in which substantially all the repeating units are derived from one or two cycloolefins; however, they can also be present as part of a block or random copolymer which also contains units derived from one or more copolymerisable monomers, eg an acyclic olefin. The cycloolefin can be a substituted or unsubstituted monocyclic or multicyclic (including dicyclic) monoolefin or multi-olefin (including diolefin), eg cyclo-pentene, cycloheptene, cyclooctene, cyclododecene, 1,5-cyclooctadiene, 1,5,9-cyclododecadiene, norbornene, norbornadiene, ethylidene norbornene and dicyclopentadiene. The preparation and properties of cycloolefin polymers is described in "The Stereo Rubbers", edited by Saltman and pubished by John Wiley and Sons (1977), pages 285 to 364, the disclosure of which is incorporated herein by reference. These polymers are generally characterized by a low melting point (usually in the range of 0° to 80°C) which can to some extent be controlled by regulating the *cis/trans* ratio (and hence the crystallinity) of the polymer; the *trans* content of the polymer is generally at least 60%, eg 60 to 90%. The polymer retains cyclic rings in its structure, and these rings may be interlocked, eg as in the catenanes or nectinodanes. Particularly good results have been obtained in the present invention using polyoctenamer with a *trans* content of 60 to 90% (melting point about 30°C to about 70°C).

The polymer component can contain units derived from one or more monomers which are not cycloolefins. Such units can be present as part of a copolymer with a cycloolefin, but are preferably present in a second polymer which is free from units drived from a cycloolefin. The second polymer may be crystalline, for example a polymer of one or more acyclic substituted or unsubstituted olefins, a polyester or a poly-actone; if present, such as a polymer can be present in amount, for example, of 10 to 50% by weight of the total polymer component. Alternatively, the second polymer can be amorphous, preferably an elastomer, eg. polybutadiene or an ethylene/propylene/diene polymer (EPDM); such a polymer can be present in amount, for example, of 5 to 15% by weight of the total polymer component, and frequently serves to improve the melt processibility of the composition, and/or its physical properties after processing.

The particulate conductive filler preferably consists essentially of carbon black, particularly one which has a particle size (D) of 20 to 150 millimicrometer and a surface area (S) in $m^2/g$ such that the ratio S/D is not more than 10. However, the invention includes the use of other conductive fillers in place of all or part of the carbon black. The amount of conductive filler should be such that the composition has the desired resistivity, usually 1 to $5 \times 10^5$ ohm.cm, preferably $10^3$ to $10^5$ ohm.cm for heaters powered by line voltages, eg. of 120 or 240V AC, and preferably 10 to 1000 ohm.cm. for heaters powered by battery voltages, eg. 4 to 48 volts.

The conductive polymer composition preferably also contains an antioxidant, preferably in amount 0.5 to 4%, particularly 1 to 3%, by volume, based on the volume of the polymeric component. The melting point of the antioxidant is preferably below the temperature at which the polymers processed, eg. below 100°C, preferably 40° to 100°C. Particularly good results have been obtained using antioxidants which contain a hindered phenol group, preferably a 1,3-di-t-butyl-2-hydroxy phenyl group; which preferably have a molecular weight greater than 400, particularly greater than 600; and which may or may not contain a thio, ester, amino or other functional group; for example the antioxidants of this type sold by Ciba Geigy under the trade name Irganox. The presence of the antioxidant is important in preventing degradation of the polymer during processing and in ensuring that there is not an excessive change in the resistivity of the composition when it is subjected to temperatures in the operating range of the device, eg. 0° to 70°C.

The conductive polymer composition can also contain conventional ingredients such as nonconductive fillers, processing aids, pigments and fire retardants, and, when the composition is to be cross-linked chemically, suitable cross-linking agents.

The conductive polymer composition is preferably shaped by melt-extrusion, molding or another melt-shaping operation. It is important to avoid excessive mixing time or intensity, which can cause the product to have too high a resistivity. It is also possible to process the composition in the form of a solution, eg. by casting a solution of the conductive polymer which is then allowed to dry. If desired, the polymer can be lightly cross-linked before it is shaped eg. by irradiation to a dose of 1 to 6 Mrad.

After the composition has been shaped, it is preferably cross-linked. Cross-linking is preferably effected by radiation, eg. by an electron beam, to a dosage which adequately cross-links but does not deleteriously degrade the polymeric component, eg. in the range 2 to 100 Mrad, preferably 3 to 30 Mrad,

3

# EP 0 138 424 B1

eg. 5 to 15 Mrad. Alternatively, cross-linking can be effected with the aid of a chemical cross-linking agent, eg. a peroxide such as dicumyl peroxide or sulfur. In the absence of such cross-liking, the PTC element may become very brittle, thus severely limiting the practical utility of the device. If the composition is not cross-linked within a relatively short time after processing eg. within a week or two, it becomes progressively more brittle. However, if the shaped composition is reheated, its flexibility can be restored. The temperature increase required is not large and can be provided in a suitable cross-linking step, eg. by using a suitably high rate of radiation, and the cross-linking will preserve the flexibility permanently.

The PTC element and the electrodes can be of any shape and dimension which results in a useful device, for example as disclosed in the publications referenced above, especially a heater or a circuit protection device. The electrodes can be in physical contact with the PTC element or physically separated therefrom by a conductive layer, eg. of a conductive polymer composition exhibiting ZTC behavior, preferably a ZTC conductive polymer based on a substantially amorphous polycycloolefin.

The present invention provides greatly improved heaters which will self-regulate at a temperature in the range of −20° to 70°C. Furthermore, by selecting a suitable cycloolefin and if necessary adjusting the cis/trans ratio of the polymer, the $T_s$ of the heater (and hence the substrate temperature which it will maintain) can be adjusted according to the particular end result desired. For example, for freeze protection, a heater having a $T_s$ of 0 to 40°C, eg. 5 to 25°C, is particularly useful. For heating the human body, a $T_s$ of 20 to 50°C is desirable, since the surface temperature of the heater should be less than about 45°C to ensure that the skin is not thermally injured. Furthermore, the conductive polymer compositions used in the heaters (and other devices) of the invention not only have good resistivity/temperature characteristics, but also can be processed by conventional techniques, can be cross-linked to give satisfactory mechanical properties, in particular flexibility and toughness, and have good resistance stability. Earlier attempts to make such heaters have not been wholly successful. For example, PTC compositions based on cis-1,4-polybutadiene do not increase sufficiently in resistivity when heated; poly-e-caprolactone has a melting point (about 65°C) which is too high for many purposes and has poor physical properties, especially above the melting point; and compositions comprising low-melting waxes are not resistance-stable and have poor physical properties.

Substrates which can be heated with the electric links according to the invention include human beings, for example those who must remain in cold environments such as divers, cold room workers, soldiers, airmen and hunters; those who are ill, for example victims of hypothermia or burn injury; and newly born children. Electrical devices in accordance with the invention may be used in deep sea diving suits, articles of human apparel, eg. boots and other footwear, gloves and clothing, and other articles which can be used to heat human beings, eg. seats (for example in cars and other vehicles), mattresses and heating pads, which incorporate PTC heaters as defined above. Other mammals can also be heated in accordance with the invention, for example newly born chickens. The heaters of the invention can also be used to heat blood and other fluids wich are to be given to a human being, eg. in a blood transfusion. They can also be used to heat diesel fuel, for example in buildings and vehicles, and other fuels, for example in spacecraft. They can also be used to heat electronic components, including those package in a plastic.

The electrical devices are also useful when current does not pass through the composition, for example when used as stress-grading layers in electrical equipment, eg. high voltage cables.

The invention is illustrated by the following Examples, which are summarized in Tables 1 and 2 below. In each of the Examples, the ingredients and amounts thereof indicated in the Tables were used (note that the amounts are in parts by volume in Table 1 and in parts by weight in Table 2).

In Examples 1 to 8, the ingredients were melt-blended in a Brabender, and the blend was compression molded into a plaque. The plaque was then irradiated to the dosage shown in the Table. Silver paint electrodes were placed on the plaque, and the change in resistivity of the composition with temperature was determined. Each of the compositions exhibited a good PTC effect.

In each of Examples 9, 10 and 11, the ingredients were fed at metered rates to the barrel of twin screw extruder fitted with a cross-head die, and the resulting mixture was melt extruded around a pair of parallel wire electrodes to form a strip heater of conventional cross-section, which was then cross-linked by radiation to the dose shown.

In each of Examples 12, 13, 14 and 16, the ingredients were melt-blended on a Banbury, and the blend was diced and melt extruded around a pair of parallel wire electrodes to form a strip heater of conventional cross-section, which was then cross-linked by radiation to the dose shown.

In Example 15, the ingredients were dry-blended and then dumped in the hopper of a single screw extruder through which the mixture was melt-extruded around a pair of parallel wire electrodes to form a strip heater of conventional cross-section, which was then cross-linked by radiation to the dose shown.

The various ingredients given in the Tables are further identified below.

Vestenamer 8012 is sold by Hüls and is a polymer of cyclooctene having a trans content of about 80%, a crystalline melting point of about 55°C, and a crystallinity of about 11% (corresponding to a crystallinity of about 32% measured by DSC by reference to 100% crystalline polycyclooctene). In Examples 9 and 10, as indicated by the asterisk, the polymer was pre-irradiated to a dose of 5 Mrad.

Petrothene NA—344 is a low density polyethylene sold by ICI.

DYNH—1 is a low density polyethylene sold by Union Carbide.

DPD—6169 is an ethylene/ethyl acrylate copolymer sold by Union Carbide.

4

*Vistalon 1721* is an ethylene/propylene rubber sold by Exxon.

*TPR—5490* is a thermoplastic rubber sold by Reichhold Chemical.

*DHDA—7704* is sold by Union Carbide and is a dispersion of a carbon black (about 38% by weight) in an ethylene/ethyl acrylate copolymer.

*Statex G* is a carbon black sold by Columbian Chemicals.

TABLE 1

| | EXAMPLE NO. | | | | | | | | | | |
|---|---|---|---|---|---|---|---|---|---|---|---|
| | 1 | 2 | 3 | 4 | 5 | 6 | 7 | 8 | 9 | 10 | 11 |
| Vestenamer 8012 | 99 | 79 | 66 | 79 | 66 | 74 | 66 | 89 | 99* | 84* | 84 |
| DPD 6169 | - | - | - | 20 | 33 | - | - | - | - | - | - |
| DYNH-1 | - | 20 | 33 | - | - | - | - | 10 | - | 15 | 15 |
| Vistalon 1721 | - | - | - | - | - | 25 | 33 | - | - | - | - |
| Statex G | 25 | 25 | 25 | 25 | 25 | 25 | 25 | 25 | 25 | 25 | 25 |
| Irganox 1035 | 1 | 1 | 1 | 1 | 1 | 1 | 1 | 1 | 1 | 1 | 1 |
| Dose (Mrad) | 10 | 10 | 10 | 10 | 10 | 10 | 10 | 10 | 10 | 10 | 10 |
| Log Resistivity (ohm.cm) at 20° C | 2 | 3 | 2.5 | 2 | 2.5 | 3 | 3 | 3.5 | | | |

TABLE 2

| | EXAMPLE NO. | | | | |
|---|---|---|---|---|---|
| | 12 | 13 | 14 | 15 | 16 |
| Vestenamer | 83 | 69.5 | 21 | 43 | 30 |
| Petrothane NA-344 | - | 15 | - | - | 10 |
| TPR-5490 | - | - | 63 | - | - |
| DHDA 7704 | - | - | - | 56 | 58 |
| XC-72 | 16 | 14.5 | 12.4 | - | |
| Black Pearls 2000 | - | - | 2.6 | - | |
| Irganox 1035 | 1 | 1 | 1 | 1 | 2 |
| Mrad | 5 | 5 | 5 | 5 | 5 |

**Claims**

1. An electrical device which comprises
(1) a PTC element which is composed of a conductive polymer composition which exhibits PTC behavior and which comprises
(a) a polymer component which comprises at least 15% by weight of repeating units derived from a cycloolefin, which has a crystallinity of at least 5% as measured by differential scanning calorimetry using high density polyethylene as the standard, and which has a crystalline melting point in the range of 0° to 80°C, and
(b) a particulate conductive filler which is dispersed in said polymer component; and
(2) at least two electrodes which can be connected to a source of electrical power to cause current to pass through the PTC element.
2. A device according to claim 1 wherein the polymer component comprises at least 50% by weight of a cycloolefin polymer in which at least 80% by weight of the repeating units are derived from at least one cycloolefin.
3. A device according to claim 1 or 2 wherein the polymer component comprises a blend of 50 to 90% by weight of said cycloolefin polymer and 10 to 50% by weight of an acyclic olefin polymer.
4. A device according to claim 1, 2 or 3, wherein the polymer component comprises a polymer of cyclo-octenamer having a trans content of 60 to 90%.
5. A device according to any one of the preceding claims wherein the polymer component has been cross-linked.
6. A device according to claim 5 wherein the polymer component has been cross-linked by radiation to a dosage of 5 to 30 Mrad.
7. A device according to any one of the preceding claims which further comprises an antioxidant in the polymer which has a melting point of 40° to 100°C.
8. A device according to claim 7, wherein the antioxidant is a hindered phenol and has a molecular weight of at least 600.
9. An electric heater comprising a device according to any one of the preceding claims.

**Patentansprüche**

1. Elektrische Einrichtung, umfassend
(1) ein PTC-Element, das aus einer leitfähigen Polymerzusammensetzung besteht, die ein PTC-Verhalten zeigt und die folgendes aufweist:
(a) eine Polymerkomponente, die mindestens 15 Gew.-% an Repetiereinheiten enthält, die von einem Cycloolefin abgeleitet sind, das einen Kristallisationsgrad von mindestens 5% hat, gemessen durch Differentialscanningkalorimetrie unter Verwendung von Niederdruckpolyethylen als Standard, und das einen Kristallschmelzpunkt im Bereich von 0°C bis 80°C hat, und

(b) einen teilchenförmigen leitfähigen Füllstoff, der in der Polymerkomponente fein verteilt ist; und

(2) mindestens zwei Elektroden, die an eine elektrische Energiequelle anschließbar sind, um durch das PTC-Element Strom fließen zu lassen.

2. Einrichtung nach Anspruch 1, wobei die Polymerkomponente mindestens 50 Gew.-% eines Cycloolefinpolymers aufweist, bei dem mindestens 80 Gew.-% der Repetiereinheiten von mindestens einem Cycloolefin abgeleitet sind.

3. Einrichtung nach Anspruch 1 oder 2, wobei die Polymerkomponente eine Mischung von 50 bis 90 Gew.-% des Cycloolefinpolymers und 10 bis 50 Gew.-% eines acyclischen Olefinpolymers aufweist.

4. Einrichtung nach Anspruch 1, 2 oder 3, wobei die Polymerkomponente ein polymers Cyclooctenamer aufweist, das einen trans-Gehalt von 60 bis 90% hat.

5. Einrichtung nach einem der vorherigen Ansprüch, wobei die Polymerkomponente vernetzt worden ist.

6. Einrichtung nach Anspruch 5, wobei die Polymerkomponente durch Bestrahlung bis zu einer Dosis von 5 bis 30 Mrad vernetzt worden ist.

7. Einrichtung nach einem der vorherigen Ansprüche, die außerdem ein Antioxidans in dem Polymer aufweist, das einen Schmelzpunkt von 40°C bis 100°C hat.

8. Einrichtung nach Anspruch 7, wobei das Antioxidans ein behindertes Phenol ist und ein Molekulargewicht von mindestens 600 hat.

9. Elektrische Heizeinrichtung, umfassend eine Einrichtung nach einem der vorherigen Ansprüche.

**Revendications**

1. Dispositif électrique qui comprend

(1) un élément CTP qui est consituté d'une composition polymérique conductrice qui présente un comportement CTP et qui comprend

(a) un constituant polymérique qui renferme au moins 15% en poids de motifs répétés dérivés d'une cyclooléfine, qui possède une cristallinité d'au moins 5%, telle qu'elle est mesurée par calorimétrie par analyse différentielle en utilisant comme substance de référence du polyéthylène haute densité, et qui possède un point de fusion cristalline compris dans l'intervalle de 0° à 80°C , et

(b) une charge conductrice en particules qui est dispersée dand ledit constituant polymérique; et

(2) au moins deux électrodes qui peuvent être reliées à une source de courant électrique pour provoquer le passage du courant à travers l'élément CTP.

2. Dispositif suivant la revendication 1, dans lequel le constituant polymérique comprend au moins 50% en poids d'un polymère cyclo-oléfinique dans lequel au moins 80% en poids des motifs répétés sont dérivés d'au moins une cyclo-oléfine.

3. Dispositif suivant la revendication 1 ou 2, dans lequel le constituant polymérique comprend un mélange de 50 à 90% en poids du polymère cyclo-oléfinique et de 10 à 50% en poids d'un polymère d'oléfine acyclique.

4. Dispositif suivant la revendication 1, 2, ou 3, dans lequel le constituant polymérique comprend un polymère de cyclo-octènamère ayant une teneur en isomère trans de 60 à 90%.

5. Dispositif suivant l'un quelconque des revendications précédentes, , dans lequel le constituant polymérique a été réticulé.

6. Dispositif suivant la revendication 5, dans lequel le constituant polymérique a été réticulé par radiation à une dose de 5 à 30 Mrads.

7. Dispositif suivant l'une quelconque des revendications précédentes, qui comprend en outre un antioxydant dans le polymère qui possède un point de fusion de 40° à 100°C.

8. Dispositif suivant la revendication 7, dans lequel l'anti-oxydant est un phénol à encombrement stérique et possède un poids moléculaire d'au moins 600.

9. Dispositif électrique de chauffage comprenant un dispositif suivant l'une quelconque des revendications précédentes.